# EUROPEAN PATENT SPECIFICATION

(11) **EP 0 674 904 B1**
(45) Date of publication and mention of the grant of the patent: **18.08.1999**
(21) Application number: 95302003.9
(22) Date of filing: 24.03.1995
(51) Int. Cl.: A61K 31/665

(54) **Use of phosphoric acid diester compounds for suppressing hepatic metastases of tumors**
Verwendung von Phosphorsäurediester-Verbindungen zur Unterdrückung von Krebsmetastasen in der Leber
Utilisation des composés de diester d'acide phosphorique pour la suppression des métastases du cancer du foie

(30) Priority: 29.03.1994 JP 5867194; 04.10.1994 JP 24016794
(43) Date of publication of application: 04.10.1995
(73) Proprietor: Senju Pharmaceutical Co., Ltd., Osaka-shi, Osaka 541-0046 (JP)
(72) Inventor: Toge, Tetsuya, Hiroshima 733 (JP); Hirai, Toshihiro, Hiroshima-shi Hiroshima 731-51 (JP); Yoshimoto, Akihiro, Hiroshima-shi Hiroshima 734 (JP); Yoshida, Kenichi, Ibaraki-shi Osaka 567 (JP)
(74) Representative: Burford, Anthony Frederick

(56) References cited:
- DATABASE WPI Week 8731 Derwent Publications Ltd., London, GB; AN 87-218325 & JP-A-62 145 019 (SENJU SEIYAKU KK) , 19 December 1985
- ARZNEIMITTELFORSCHUNG, vol. 42, no. II, 1992 pages 1072-1074, KURIBAYASHI, Y. ET AL 'IN VITRO STUDIES ON THE INFLUENCE OF L-ASCORBIC ACID 2-(3,4-DIHYDRO-2,5,7,8-TETRAMETHYL-2-(4,8, 12-TRIMETHYLTRIDECYL)-2H-1-BENZOPYRAN-6-YL -HYDROGEN PHOSPHATE) POTASSIUM SALT ON LIPID PEROXIDATION AND PHOSPHOLIPASE A2 ACTIVITY'
- PROCEEDINGS OF THE ANNUAL MEETING OF THE AMERICAN ASSOCIATION FOR CANCER RESEARCH, vol. 34, March 1993 pages 570-571, KOHN, E.C. ET AL 'SIGNAL TRANSDUCTION THERAPY OF METASTASIS'
- PATENT ABSTRACTS OF JAPAN vol. 014 no. 543 (C-0783) ,30 November 1990 & JP-A-02 229168 (TAKEDA CHEM IND LTD) 11 September 1990,
- PATENT ABSTRACTS OF JAPAN vol. 014 no. 543 (C-783) ,30 November 1990 & JP-A-02 229169 (TAKEDA CHEM IND LTD) 11 September 1990,

## Description

The present invention relates to useful suppressory compositions against hepatic metastasis of tumors. More specifically, this invention relates to suppressory compositions against hepatic metastasis of tumors which comprise a phosphoric acid diester compound of ascorbic acid and a tocopherol or a pharmacologically acceptable salt thereof.

At present, remarkable progress has been made in the treatment of malignant tumors (cancers) in terms of pharmacotherapy, surgical therapy, radiation therapy, etc., but no effective preventive measure is available against cancer metastasis, or an indicator of cancer malignancy. In cancer treatment, therefore, the development of an improved suppressory composition against cancer metastasis is highly desired. Cancer metastasis is roughly classified into hematogenous metastasis and lymphatic metastasis, and the establishment of cancer metastasis proceeds through the sequential steps of (1) infiltrative and destructive proliferation of tumor cells at the primary site, (2) segregation of tumor cells or cell colonies, (3) migration of tumor cells, (4) fixation or establishment and (5) proliferation at the site of fixation. Cancer metastasis is involved particularly in such organs as the lung, liver and digestive organs, and the present Inventors, with a specific object of developing an improved cancer-metastasis suppressory composition, have conducted extensive research studies on the mechanism of cancer metastasis, inclusive of pulmonary metastases of cancers.

In a series of such research studies, the present Inventors carried out investigation into the hepatic metastasis of malignant tumors. Intensive research was performed on the basis of the hypothesis that the level of intrahepatic or liver lipid peroxide (LPO) would rise due to surgical invasion, or stress, and in turn damage the vascular endothelia, resulting in enhanced hematogenous metastasis to the liver of malignant tumors. As a result, it was revealed that surgical invasion promotes the hepatic metastasis of tumors and elevates the level of liver lipid peroxide. This novel finding was followed by further research to seek an improved suppressory composition against hepatic metastasis of tumors. It was found that a kind of phosphoric acid diester compound suppresses effectively not only elevation of the level of liver lipid peroxide but also nodulation of tumors on the liver surface and provides a promising suppressory composition against hepatic metastasis of malignant tumors.

The present invention provides the use of a phosphoric acid diester compound represented by the following formula (I): 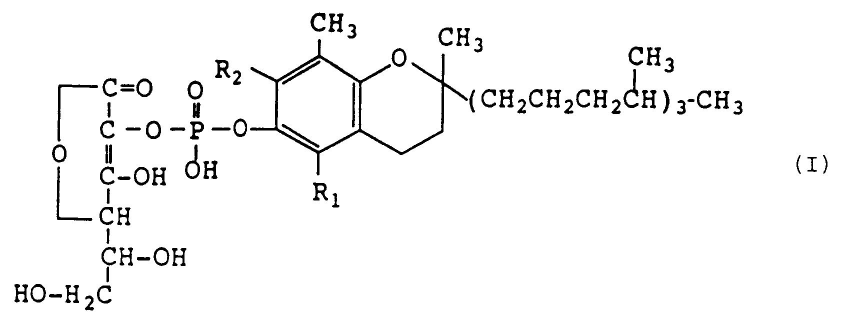 (wherein R₁ and R₂ each is the same as or different from the other and represents hydrogen or methyl), or a pharmacologically acceptable salt thereof (hereinafter referred to collectively as "Present Compounds") in the manufacture of a medicament for suppressing hepatic metastasis of tumors.

The Present Compounds can suitably be synthesized, by, for example, methods such as those described in JP-A-2-44478 (1990) and JP-A-62-205091 (1982).

The Present Compounds are already known to have application in a wide variety of fields, such as anti-cataract agents, prophylactic and therapeutic agents for climacteric disorders, as cosmetics exhibiting skin-beautifying activity (JP-A-2-44478 (1990)), as anti-inflammatory agents (JP-A-1-27044) and as antiulcer agents (JP-A-63-270626 (1988)) and as prophylactic and therapeutic drugs for ischemic diseases of the organs (JP-A-2-111722 (1990)). It also was known that the potassium salt of L-ascorbic acid 2-[3,4-dihydro-2,5,7,8-tetramethyl-2-(4,8,12-trimethyl(tridecyl)-2H-1-benzopyran-6-yl-hydrogen phosphate ("EPC-K₁") inhibits lipid peroxidation and phospholipase A₂ activity (Y. Kuribayaschi et al (Arzneim. Forsch./Drug Res. 42 (II) (1992) 1072-1074)). However, it was not previously known that the Present Compounds are useful as suppressory agents against hepatic metastasis of tumors.

JP-A-2-229168 discloses that pyrazolin-5-one compounds substituted at the 1-position by an (optionally substituted) C₈-C₂₀ chain hydrocarbon group or an (optionally substituted) polyether chain of 8 to 20 carbon and oxygen atoms and substituted at the 3-position by an (optionally substituted) methyl or cyclic group are peroxylipid formation, lipoxygenase, phospholipid or collagenase inhibitors. They are reported to be useful in the treatment of diseases of the circulatory organs, inflammations, allergies etc., and cancer metastasis.

E.C. Kohn et al (Proc. Am. Ass. Cancer Res. 34 (1993) 570-571) reports the effect of certain molecular modifications of carboxyamidotriazole ("CAI") on the inhibition of calcium influx, arachidonic acid release and tumor cell proliferation. The results are stated to provide a link between calcium influx, calcium mediated arachidonic acid release, tumor proliferation, and metastasis. They state that their studies further strengthen the previously reported tie between calcium influx and phospholipase A₂ activation.

The Present Compounds can suitably be utilized in the form of either the free acids or pharmacologically acceptable salts for the objective of this invention. As pharmacologically acceptable salts, there may be exemplified alkali metal salts, such as sodium and potassium salts, and alkaline earth metal salts, such as calcium and magnesium salts. However, any other pharmacologically acceptable salts can be used.

Suppressory compositions against hepatic metastases of tumors can be formulated with one or more of the Present Compounds depending upon the intended purpose and necessity.

The Present Compounds exhibit extremely low toxicity and good safety. For example, the potassium salt of a phosphoric acid diester of L-ascorbic acid and DL-α-tocopherol (hereinafter referred to briefly as "EPC-K") shows LD₅₀ values of 5 g/kg p.o. (in rats) and not less than 100 mg/kg i.v. (in rats).

The Present Compounds can suitably be used orally or parenterally (for example, by way of intravenous injection, subcutaneous injection, intramuscular injection and drip infusion). They can suitably be processed and formulated by conventional methods in either solid dosage form such as tablets, granules, powders and capsules or liquid preparations, such as injectable solutions. These pharmaceutical preparations may suitably incorporate a variety of conventionally used additives, such as excipients, binders, disintegrating agents, dispersants, reabsorption promoters, buffers, surfactants, solubilizers, preservatives, emulsifiers, isotonizing agents, stabilizers and pH regulating agents.

Doses of the Present Compounds required to suppress hepatic metastases of tumors vary depending upon the actual compound(s) used, the age, body weight and sex of the patient and the symptoms of diseases as well as the type of dosage form. Usually, the Present Compounds are desirably administered to human adults at a single dose in the range of 0.5 to 200 mg, preferably 2 to 50 mg, once a day in the case of injectable solutions, and at a single dose in the range of 5 to 2,000 mg, preferably 20 to 500 mg, several times a day in the case of preparations for internal use.

Unless contraindicated, the Present Compounds may be used with other suppressory compounds against hepatic metastasis of tumors and/or other active agents capable of producing different medicinal effects.

The following Examples and Preparation Examples illustrate this invention in more detail, but the present invention is limited by these Examples. In the accompanying drawings:
Fig. 1 is a graph showing the numbers of tumor nodules on the liver surface (plotted on the ordinate) as found in the group undergoing laparothoractomy (Group LT), group undergoing laparotomy (Group L) and control group (Group C), respectively;
Fig. 2 is a graph showing changes in level of liver lipid peroxide (LPO, nmol/g; plotted on the ordinate) as found in the group undergoing laparothoractomy (Group LT), group undergoing laparotomy (Group L) and control group (Group C), respectively;
Fig. 3 is a graph showing levels of liver lipid peroxide (LPO, nmol/g; plotted on the ordinate) as determined 24 hours after surgical invasion (laparothoractomy) inflicted in the group not treated by administration of a Present Compound (Group C) and group treated by administration of EPC-K (Group EPC-K), respectively; and
Fig. 4 is a graph showing the numbers of tumor nodules on the liver surface (plotted on the ordinate) as determined 3 weeks later in the group not treated by administration of a Present Compound (Group C) and group treated by administration of the EPC-K (Group EPC-K), respectively.

### Example 1

### Experiments on hepatic metastasis of tumor in relation to a level of liver lipid peroxide:

Using a model of hepatic metastasis of tumor, an experiment was conducted to determine the correlation of hepatic metastasis of tumor with the level of liver lipid peroxide (LPO). In addition, an experiment was conducted to determine the suppressory effect of EPC-K on liver lipid peroxide (LPO) and on hepatic metastasis of tumor.

### Method:

Male Donryu rats (ca. 10-weeks old) were employed as the experimental animal, while AH60C cells (derived from rat hepatoma) were used as the tumor cell. The rats were divided into three groups, namely a laparothoractomy group (Group LT) undergoing intratracheal intubation, followed by thoraco-laparotomy for one hour, a laparotomy group (Group L) undergoing only laparotomy for one hour and a control group (Group C) undergoing laparotomy, followed by immediate closure of the abdomen. The rats were subjected to laparotomy under anesthesia with ether and Ketalar and given 5 x 105 of AH60C cells through the portal vein to inflict surgical invasion, followed by laparotomy three weeks later to determine the number of tumor nodules on the liver surface. In addition to this, the serum samples were drawn, while the liver and lung tissues were isolated, on Disease Days 1, 2 and 3 to determine their respective levels of lipid peroxide by the TBA method.

Then, the LT group was subdivided into a group treated an hour before surgical invasion by intravenous injection of EPC-K at a dose of 5 mg/kg (Group EPC-K) and a group not treated with a Present Compound (Group C) to determine levels of liver lipid peroxide 24 hours later as well as numbers of tumor nodules on the liver surface 3 weeks later. Test of data was done by Mann-Whitney U method.

### Results:

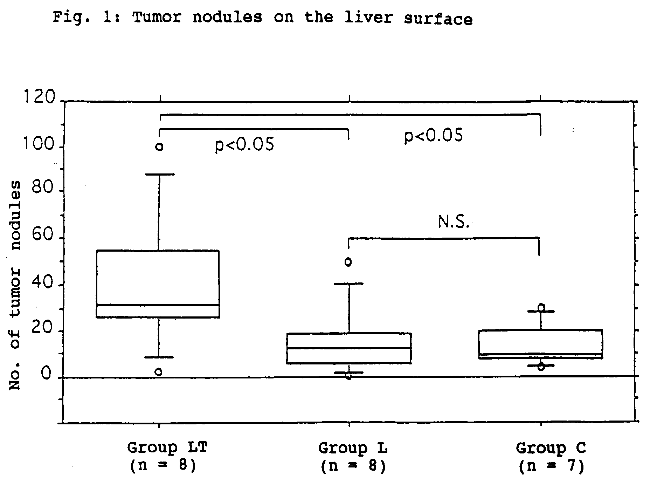

The number of tumor nodules on the liver surface was found to be 40.6 ± 29.7 in Group LT, 15 ± 15.8 in Group L and 13.7 ± 9.4 in Group C, respectively (Refer to Fig. 1). Group LT showed significant difference (p < 0.05) against Groups L and C.

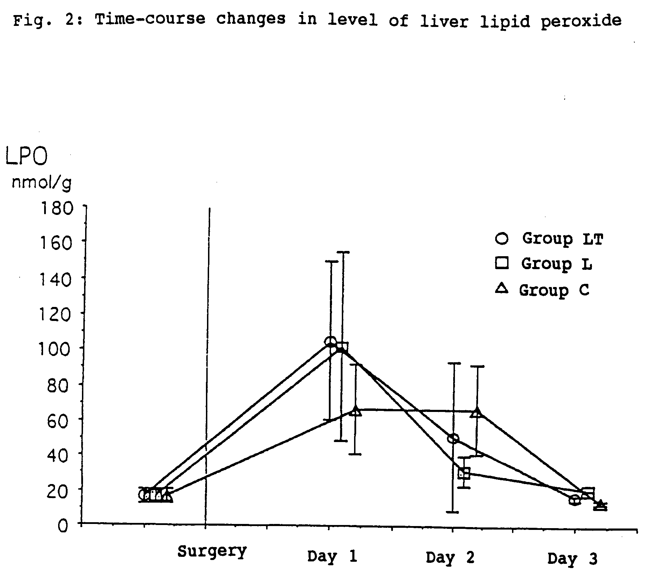

The level of liver lipid peroxide was found to be 16.6 ± 3.8 nmol/g (n = 4) in Group C, which figure was taken as a prior value. The surgical invasion raised the levels of intrahepatic lipid peroxide, which reached a peak on Disease Day 1; 93.9 ± 22.5 nmol/g in Group LT, 102.3 ± 53.6 nmol/g in Group L and 66.9 ± 25.5 nmol/g in Group C (refer to Fig. 2). There was no significant difference between Groups LT and C, but Group LT displayed a tendency toward elevated level of liver lipid peroxide. On Disease Day 3, the levels were found in three groups to return nearly to the prior value. However, the lipid peroxide levels in the serum and lung remained unchanged, with no difference being noted among these three animal groups.

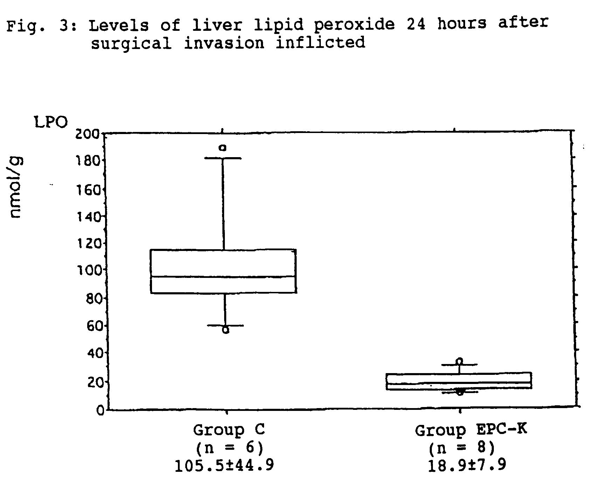

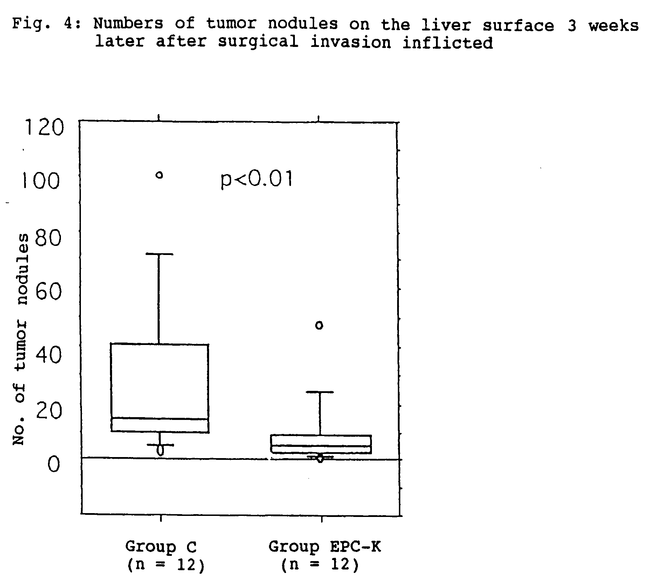

Prior administration of EPC-K at a dose of 5 mg/kg caused the level of liver lipid peroxide 24 hours later to decrease to 18.9 ± 7.9 nmol/g, showing to significantly suppressed elevation of the level (p < 0.05) (refer to Fig. 3), and reduced the numbers of tumor nodules on the liver surface and to 8.9 ± 12.7 in Group EPC-K compared with 27.2 ± 30.0 in Group C (not treated), showing significant suppression in Group EPC-K (p < 0.01) (refer to Fig. 4).

### Conclusion:

Surgical invasion, a kind of stress, raised the number of tumor nodules on the liver surface (hepatic metastasis) and also increased the level of liver lipid peroxide. EPC-K suppressed both the elevation in level of liver lipid peroxide and the increase in tumor nodulation on the liver surface and constitutes a prospective suppressory agent against hepatic metastasis of tumors.

| Preparation Example 1: | Tablet for internal use: |
|---|---|
| EPC-K | 100 mg |
| Lactose | 75 mg |
| Starch | 20 mg |
| Polyethylene glycol 6000 | 5 mg |

The above-described ingredients are mixed and processed into a tablet by conventional tableting procedure. Sugar coating may be provided to the tablet if necessary.

| Formulation Example 2: | Injectable solution: |
|---|---|
| EPC-K | 200 mg |
| Mannitol | 5.0 g |
| 1N-Sodium hydroxide | q.s. |
| Distilled water | to 100 ml (pH 6.5) |

The above-described ingredients are mixed and sterile-filtered by the conventional procedure. The filtrate is filled sterile in 5 ml portions into glass ampoules, followed by fusing to give ampoules containing an injectable solution.

## Claims

1. The use of a phosphoric acid diester compound of the formula: 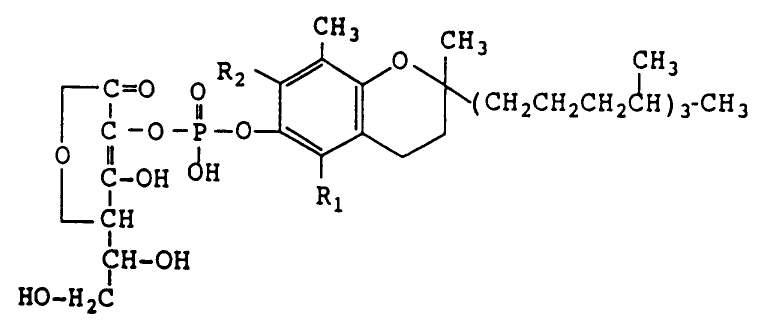 (wherein R₁ and R₂ each is the same as or different from the other and represents hydrogen or methyl) or a pharmacologically acceptable salt thereof in the manufacture of a medicament for suppressing hepatic metastasis of tumors.

2. A use as claimed in Claim 1, wherein the medicament is for enteral administration.

3. A use as claimed in Claim 2, wherein the medicament is for administration at a unit dose of 20 to 500 mg.

4. A use as claimed in Claim 1, wherein the medicament is for parenteral administration.

5. A use as claimed in Claim 4, wherein the medicament is an injectable solution.

6. A use as claimed in Claim 5, wherein the medicament is for administration at a unit dose of 2 to 50 mg.

7. A use as claimed in any one of the preceding claims, wherein the phosphoric acid diester is the phosphoric acid diester of L-ascorbic acid and DL-2-tocopherol.

## Patentansprüche

1. Verwendung einer Phosphorsäurediester-Verbindung mit der Formel: 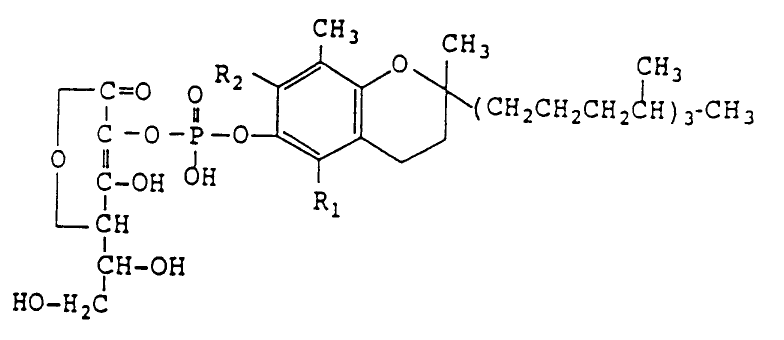 (wobei R₁ und R₂ jeweils dasselbe ist oder vom anderen verschieden ist und Wasserstoff oder Methyl darstellt) oder eines pharmakologisch annehmbares Salzes derselben bei der Herstellung eines Medikaments zum Unterdrücken von Metastasen von Tumoren in der Leber.

2. Verwendung nach Anspruch 1, wobei das Medikament für enterale Applikation bestimmt ist.

3. Verwendung nach Anspruch 2, wobei das Medikament für eine Applikation mit einer Dosiseinheit von 20 bis 500 mg bestimmt ist.

4. Verwendung nach Anspruch 1, wobei das Medikament für parenterale Applikation bestimmt ist.

5. Verwendung nach Anspruch 4, wobei das Medikament eine injizierbare Lösung ist.

6. Verwendung nach Anspruch 5, wobei das Medikament für die Applikation mit einer Dosiseinheit von 2 bis 50 mg bestimmt ist.

7. Verwendung nach einem der vorhergehenden Ansprüche, wobei der Phosphorsäurediester der Phosphorsäurediester von L-Ascorbinsäure und DL-2-Tocopherol ist.

## Revendications

1. Utilisation d'un composé diester d'acide phosphorique de formule 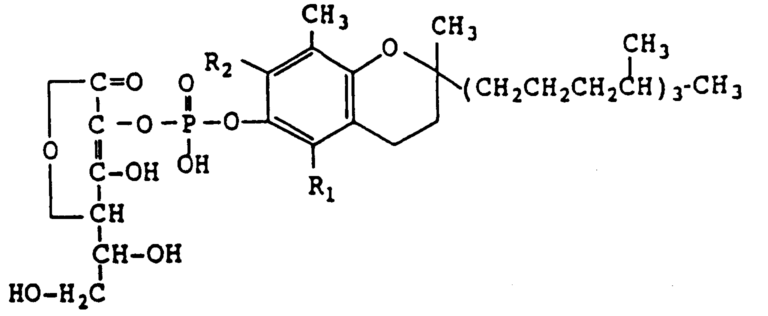 (dans laquelle R₁ et R₂ sont chacun identiques ou différents l'un de l'autre et représentent des atomes d'hydrogène ou des groupements méthyle) ou d'un de ses sels pharmaceutiquement acceptable pour la fabrication d'un médicament supprimant les métastases hépatiques des tumeurs.

2. Utilisation selon la revendication 1, dans laquelle le médicament est destiné à une administration entérique.

3. Utilisation selon la revendication 2, dans laquelle le médicament est destiné à une administration à une dose unitaire de 20-500 mg.

4. Utilisation selon la revendication 1, dans laquelle le médicament est destiné à une administration parentérale.

5. Utilisation selon la revendication 4, dans laquelle le médicament est une solution injectable.

6. Utilisation selon la revendication 5, dans laquelle le médicament est destiné à une administration à une dose unitaire de 2-50 mg.

7. Utilisation selon l'une quelconque des revendications précédentes, dans laquelle le diester d'acide phosphorique est le diester d'acide phosphorique de l'acide L-ascorbique et du DL-α-tocophérol.
